# EUROPEAN PATENT SPECIFICATION

(11) **EP 0 661 540 B1**
(45) Date of publication and mention of the grant of the patent: **05.08.1998**
(21) Application number: 95103930.4
(22) Date of filing: 17.01.1986
(51) Int. Cl.: G01N 33/74, G01N 33/543, G01N 33/545

(54) **Method for measuring free testosterone in biological fluids**
Verfahren zum Messen von Freitestosteronen in biologischen Flüssigkeiten
Méthode pour determiner les testosterones libres dans les fluides biologiques

(30) Priority: 04.10.1985 US 784857
(43) Date of publication of application: 05.07.1995
(62) Divisional of application: 86300336.4
(73) Proprietor: DIAGNOSTIC PRODUCTS CORPORATION, Los Angeles California 90045 (US)
(72) Inventor: Said el Shami, A. Dr. Diagnostic Products Corp., Los Angeles, Ca 90045 (US)
(74) Representative: Goldin, Douglas Michael

(56) References cited:
- EP-A- 0 015 687
- EP-A- 0 133 464
- EP-A- 0 155 104
- WO-A-85/00226
- US-A- 3 928 553

## Description

For several decades equilibrium dialysis techniques were the only available method for the measurement of free hormones in serum, and until recently were the only methods considered reliable. Equilibrium dialysis methods in this context suffer from several drawbacks including poor precision. tediousness and so on; but above all their results are highly dependent on the purity of the tracers used.

Ellis and Ekins, R. (Acta Endocr. (KbH.) Suppl. 177:106, 1973), disclosed a direct method for free hormone determinations in their paper "Direct Measurement By Radioimmunoassay of the Free Thyroid Hormone Concentration in Serium." This represented a major improvement over equilibrium dialysis methods because it allowed for the direct measurement by radioimmunoassay (RIA) of free ligand levels in serum dialysates, thus circumventing the problem of tracer purity. This method is now considered by many as the reference methodology for free hormone measurements. It is, however, still time consuming and operator-dependent, and it is unavailable to most small laboratories.

Indirect methods for the estimation of free hormone concentrations which were introduced shortly thereafter include the testosterone/steroid hormone binding globulin (SHBG) ratio, the thyroxine (T4)/thyroid binding globulin (TBG) ratio, the free T4 index (based on the product of triiodothyronine (T3) uptake and T4), and the free androgen index.

Ekins, R. (Free Thyroid Hormones; Proceedings of the International Symposium held in Venice, December 1978, Amsterdam: Excerpta Medica, 1979 72-92), introduced the concept of "direct dynamic methods" in which an anti-free ligand antibody is used in direct contact with the biological fluid during dialysis. This constitutes the basis for so-called "immunoextraction" methods.

One such method is taught in U.S. Patent No. 4,046,870 in which a two-tube immunoassay method measures the rate of transfer of T4 from binding proteins to T4-specific antibody. This method suffered from several analytical and clinical shortcomings which made it virtually just another free T4 index assay.

A second method, introduced by Clinical Assays (Cambridge, MA 02139), was a true immunoextraction method. It used a single-tube, two-stage, sequential (back-titration) technique. In this method, a serum sample is incubated with immobilized antibody; then, following a wash step, unoccupied sites on the immobilized antibody are "back-titrated" using labeled ligand. In this approach, the serum is never in contact with the labeled ligand. Although theoretically sound, it suffers from poor sensitivity and precision, and both reactions require exact timing.

Single-step immunoextraction methods for the determination of free ligand concentrations in biological specimens were the obvious next step in the development of free ligand assay systems. These methods rely on chemical rather than physical separation of labeled ligand from endogenous binders. In order to achieve this objective, several approaches can be adopted, as detailed below.

The prior art discloses that by chemically altering the structure of a given ligand, its binding to endogenous binders is reduced or diminished. This has been amply demonstrated for steroid hormones. (See the discussion of free testosterone below.) In the case of thyroid hormones, Ross, J.E. and Tapley, D.F. (Effect of various analogues on the binding of labeled thyroxine to thyroxine-binding globulin and prealbumin, Endocrinology 79:493, 1966), have shown that the binding of TBG (thyroid binding globulin) to T4 is inhibited if a fairly bulky substitution is made at the 3' position of the T4 molecule. In addition, Schall, R.F., et al (An enzyme-labeled immunoassay for the measurement of unsaturated thyroid hormone binding capacity in serum and plasma, Clin. Chem. 25:1078 (abstract) 1979), and Kleinhammer, G., et al (Enzyme immunoassay for determination of thyroxine binding index, Clin. Chem. 24:1033, 1978), independently demonstrated that TBG fails to bind to conjugates formed by labeling T4 with horseradish peroxidase. This fact constitutes the basis for the single-step immunoextraction method described in U.S. Patent No. 4,410,633 to Corning Glass Works, for the measurement of free thyroxine and free 3,5,3'-triiodothyronine wherein horseradish peroxidase is chemically attached to T4 and T3 and later radiolabeled.

In addition, the prior art also discloses that T3 and T4 require the following molecular structure for maximal binding to endogenous binding proteins, viz. TBG, thyroid binding pre-albumin (TBPA), albumin, Snyder, S.M, et al (Binding of thyroid hormones and their analogues to thyroxine-globulin in human serum, J. Biol. Chem. 251:6489, 1976); Sterling, K., et al (Equilibrium dialysis studies of the binding of thyroxine by human serum albumin, J. Clin. Invest. 41:1021, 1962):
1. The L-alanine side chain configuration;
2. The presence of 4'-hydroxyl group (primarily for TBPA and albumin binding); and
3. The presence of two (halogen) substituents in the inner and outer rings (positions 3,5,3' and 5').

Several hundred T3 and T4 analogs have been synthesized and studied for their ability to bind to thyroid hormone binding proteins.

U.S. Patent No. 4,366,143 and its European counterpart, Patent No. 00 26 103, broadly describe the use of such analogs as tracers in a single immunoextraction using simultaneous rather than sequential titration of antibody for the measurement of free hormones. (For convenience, these patents will be collectively referred to hereinafter as the "Amersham" patent.)

An intact analine side chain is required for optimal binding of T4 and T3 to TBG: the amino group on the analine side chain is the essential constituent. Analogs described in the Amersham patent are T3 and T4 molecules modified at the analine side chain. Although theoretically these analogs do not bind TBG to any significant extent, they undoubtedly bind albumin and TBPA significantly since the 4'-hydroxyl group on the T3 and the T4 molecules is left intact. It is well established that the binding of albumin and TBPA to the thyronines is quantitative, especially under physiological conditions, Sterling, K. (Molecular structure of thyroxine in relation to its binding by human serum albumin, J.Clin Invest. 43:1721, 1964), and Pages, et al (Binding of thyroxine and thyroxine analogs to human serum prealbumin, Biochem 12:2773, 1973).

The failure of the Amersham patent to recognize the importance of albumin and TBPA binding to the thyronines renders the patent's teachings inadequate for the true measurement of free T3 and free T4 in biological fluids. In fact the commercially available reagents based on the patent yield misleading and inaccurate free hormone results. This is particularly true in several pathological conditions characterized by significant alterations in the circulating albumin level.

Recent literature has shown that the albumin concentration correlates directly with free T4 concentrations generated by the Amersham assay system. In addition, it is well documented that Amersham's method consistently yields falsely decreased free T4 results in third-trimester pregnancies and in patients suffering from severe non-thyroidal illness, while yielding falsely elevated free T4 levels in cases of familial dysalbuminemic hyperthyroxinemia, a condition in which T4 is abnormally bound to circulating albumin.

During pregnancy, albumin circulates at lower than normal levels, especially during the third trimester. Since Amersham's labeled analog T4 tracer binds albumin and TBPA to a significant extent (greater than 99%), one would expect the Amersham assay system to yield lower than normal free T4 results during the third trimester: more analog tracer is available to bind T4 antibody, resulting in higher binding and lower apparent dose.

Non-esterified free fatty acids are capable of displacing labeled analog from albumin; moreover, they circulate at higher than normal concentrations during pregnancy. This could explain the lower than expected free T4 values encountered during pregnancy when assayed by the Amersham method; apparent free T4 levels would be significantly lower than expected if albumin binding to the labeled analog is substantial.

This situation is also well documented in cases of heparin therapy, where a significant elevation of non-esterified free fatty acids is present. Free T4 and free T3 levels when measured by Amersham's method on heparin-treated patients show lower than normal levels.

The same problem occurs for non-thyroidal illness, where free T3 and T4 values generated by the Amersham method have been shown to be significantly lower than for a euthyroid population, when compared to a direct equilibrium dialysis method.

The Amersham patent procedure has been found wanting by workers in the art as manifested by the observance of false and erroneous measurements of free ligand levels. Applicant has discovered that the problem stems from binding of the ligand analog tracer to certain endogenous proteins, e.g., albumin in biological fluids. I have discovered that this problem can be overcome by the use of specific chemical inhibitor reagents. This discovery represents a major advance in the art and it is believed to be deserving of a patient.

EP-A-155 104 discloses the use of a differential blocking agent in an assay of the free portion of an analyte, e.g. thyroxine, in a biological fluid in which the analyte is present partly bound to natural protein binders and in which the free analyte and an analyte derivative compete for reaction with a specific binder. The blocking agent reduces binding of the analyte derivative but not of the analyte to the natural protein binders.

### The Invention

It is an object of this invention to provide a new and improved method for measuring free testosterone ligand in biological fluids.

More particularly, the present invention has as its object the truer measurement of free testosterone ligand in biological fluids.

Briefly, this invention comprises a method for measuring the concentration of free testosterone ligand in a biological fluid in the presence of bound ligand and endogenous binding proteins, without disturbing the equilibrium between free ligand and protein-bound ligand, which method comprises
(a) incubating, in the absence of salicylate, 2,4-dinitrophenol and 8-anilino-1-naphthalenesulfonic acid, a sample of the biological fluid with (i) a ligand analog tracer which, due to its chemical structure, does not bind to some of the endogenous binding proteins but does bind to at least one other endogenous binding protein, (ii) a concentration of a specific ligand binder having an affinity constant and selectivity for the free ligand such that the equilibrium between free ligand and protein-bound ligand is not disturbed and (iii) a concentration of sulfobromophthalein (SBP) that inhibits the binding of the ligand analog tracer to said at least one other endogenous binding protein sufficient to block reaction between the ligand analog tracer and said at least one other endogenous binding protein without displacing ligand from protein-bound ligand;
(b) separating the ligand analog tracer bound to the specific ligand binder from unbound tracer; and
(c) determining the concentration of free ligand in said biological fluid.

In general, in step (c), the concentration of free ligand in said biological fluid is determined by comparing the bound fraction of ligand analog tracer in said sample to the bound fraction in a given set of free ligand calibrators. The free ligand callibrators may be prepared by adding different amounts of the ligand to ligand-free human serum, calibrating by equilibrium dialysis and assigning free ligand values.

The method is preferably carried out under physiological conditions, i.e. at about 37°C and about pH 7.4.

In general, the specific ligand binder is one which couples or binds to the free ligand and it may be a specific antibody for the free ligand or other binding agent. Appropriate specific ligand binders are known and need not be further described. The specific ligand binder may be immobilised on a solid substrate, such as polypropylene.

The ligand analog tracer is labeled in some way so as to be detectable or observable. Radiolabels are well-known and applicable, as are the other labeling means previously employed in this art, including enzymes, fluorophors, chromophores, and chemiluminescent groups integral with the ligand analog tracer molecule. The ligand analog tracer is preferably iodinated 6-hydroxytestosterone-19-carboxymethyl ether histamine.

It is well established from prior art that steroid molecules bind to their natural binders through the A and/or B ring of the molecule. See Forest, M., et al and references therein (Free and bound steroids in plasma: methodology and physiopathological implications, In: Physiological Peptides and New Trends in Radioimmunology, C.A. Bizollon, ed., Amsterdam: Elsevier/North-Holland Biochemical Press, 1981, 249-266.) Chemical alteration of the A and/or B ring will inhibit most steroids - including testosterone, progesterone, estradiol, cortisol, and so on - from binding to endogenous binders. Testosterone was selected as a representative member of this family. A testosterone analog, 6-hydroxytestosterone-19-carboxymethyl ether histamine, was systhesized and radiolabeled with iodine 125 by conventional techniques. This analog tracer was subsequently compounded in 0.01M HEPES buffer, pH = 7.4, containing 1 mg/ml charcoal-absorbed human serum albumin and 0.01% sodium azide. Blocking agents were added, as described in the specific examples below.

Antibodies to testosterone were raised in rabbits using testosterone-19-carboxymethyl ether bovine serum albumin as the immunogen, and immobilized on the inner walls of polypropylene 12x75mm tubes as described above for free T4 and free T3. Free testosterone calibrators, prepared by adding different amounts of testosterone to human serum free of any apparent testosterone, were calibrated by direct equilibrium dialysis and assigned free testosterone values in pg/ml. For the assay of free testosterone, 50 µl of calibrator or patient sample is pipetted into antitestosterone antibody-coated tubes, followed by the addition of 1.0 ml of iodinated 6-hydroxytestosterone-19-carboxymethyl ether histamine analog. The tubes are incubated for 4 hours at 37°C, then decanted and radioactivity counted. Results are computed by interpolation from the calibration curve.

### Free Testosterone Examples

Example 1: To investigate the effect of blocking agents on free testosterone results, twenty samples were assayed for free testosterone using iodinated analog - compounded as described above - both with and without sulfobromophthalein (SBP), and with various amounts of sodium salicylate, 2,4-dinitrophenol (DNP) and 8-anilino-1-naphthalenesulfonic acid (ANS). Mean values for each tracer, in pg/ml, are summarized below.

**Table 20.**

| Tracer | mg/tube: | + 5 Salicylate | + 10 Salicylate | + 0.15 DNP | + 0.3 DNP | + 1.0 ANS | + 2.0 ANS |
|---|---|---|---|---|---|---|---|
| without SBP | 7.9 (A) | 13.0 (B) | 14.3 (C) | 10.5 (D) | 13.4 (E) | 18.9 (F) | 17.7 (G) |
| with SBP | 7.1 (A') | 13.1 (B') | 14.4 (C') | 9.8 (D') | 13.5 (E') | 16.8 (F') | 15.0 (G') |

The regression equations between corresponding tracers are given below.

**Table 21.**

| | | |
|---|---|---|
| A = 1.14 | A' - 0.21 | r = 0.991 |
| B = 1.00 | B' - 0.11 | r = 0.997 |
| C = 1.02 | C' - 0.31 | r = 0.998 |
| D = 1.11 | D' - 0.30 | r = 0.996 |
| E = 1.03 | E' - 0.52 | r = 0.997 |
| F = 1.13 | F' - 0.05 | r = 0.996 |
| G = 1.19 | C' - 0.26 | r = 0.997 |
| A' = 2.76 | F' - 2.70 | r = 0.966 |
| A' = 2.34 | G' - 1.58 | r = 0.987 |

From the example above we find that the absence of sulfobromophthalein will increase the apparent free testosterone levels by 14% since sulfobromophthalein inhibits the binding of the analog tracer to albumin without displacing testosterone bound to albumin. We also find - and this is of major importance - that salicylate, 2,4-dinitrophenol and ANS displace testosterone from albumin and/or SHBG, thus increasing the apparent free testosterone as measured by this method.

Example 2: In order to check the efficacy of the analog tracer in the free testosterone assay, iodinated 6-hydroxytestosterone-19-carboxymethyl ether histamine (analog tracer) was compared to iodinated testosterone-19-carboxymethyl ether histamine (regular tracer) in assays for free testosterone in patient samples.

The tracers were compounded as described above with 10 µg/ml sulphobromophthalein. In order to maintain equivalent sensitivity, adjustments were made for each tracer in the amount of antibody immobilized onto the inner wall of the propylene tubes.

Twenty patient samples were assayed following the free testosterone protocol already described, using the two tracers mentioned above. The mean free testosterone values, in pg/ml, and the regression equation are displayed below.

**Table 22.**

| Tracer: | 6-Hydroxytestosterone-19-histamine-¹²⁵I | Testosterone-19-histamine-¹²⁵I |
|---|---|---|
| Mean (n = 20) | 11.0 (A) | 17.3 (B) |
| | A = 1.48 B + 1.24 r = 0.977 | |

The results clearly indicate that the analog 6-hydroxytestosterone-19-histamine-¹²⁵I tracer does not bind to endogenous binders, while the tracer tesetosterone-19-histamine-¹²⁵I does, thus yielding approximately 50% higher free testosterone values compared to the analog tracer under identical experimental conditions.

Example 3: To investigate the effect of sex hormone-binding globulin (SHBG) levels on the free testosterone assay system, a charcoal-absorbed human serum pool was spiked with 400 µg SHBG/milliliter, a level which is approximately 10 times normal. The SHBG-spiked pool, when assayed by the free testosterone procedure, showed a percent bound value of 99% B/Bₒ.

Since charcoal absorption removes testosterone from the serum pool, it should have free (and total) testosterone concentrations of zero - that is, percent bound values of approximately 100% B/Bₒ - both before and after spiking. The results show, as desired, that the analog tracer, 6-hydroxytestosterone-19-histamine-¹²⁵I, does not bind to even high levels of SHBG.

Example 4: In order to investigate the effect of elevated albumin levels on the free testosterone procedure, three lyophilized samples were reconstituted with aqueous solutions containing 0, 1.0, 2.0 and 3.0 gm/dl of charcoal-absorbed human serum albumin. All samples were assayed in parallel using the same tracer as in Example 3, with the following results.

**Table 23.**

| Sample | Unspiked | Spiked with Albumin (gm/dl) | | |
|---|---|---|---|---|
| | | 1.0 | 2.0 | 3.0 |
| *1* | 4.7 | 4.4 | 4.2 | 3.9 |
| *2* | 16.7 | 16.4 | 16.7 | 15.8 |
| *3* | 37.0 | 37.0 | 34.2 | 34.0 |
| *Mean* | 19.5 | 19.3 | 18.4 | 17.9 |
| *Recovery* | - | 99% | 94% | 92% |

The results show that there is no clinically significant effect due to even major increases in the albumin level. Note that samples spiked with 3.0 gm/dl represent a very high level of albumin, in the order of 7 gm/dl.

Example 5: Several patient samples were analyzed by the free testosterone procedure using the same tracer as in Example 3 both before and after charcoal absorption. Displayed below are the free testosterone concentrations (in pg/ml) before charcoal absorption, and the percent bound (%B/Bₒ) values following charcoal absorption.

**Table 24.**

| Normal Males | | Normal Females | | 3rd Trimester | |
|---|---|---|---|---|---|
| Before | After | Before | After | Before | After |
| 21.24 | 96% | 0.80 | 105% | 4.75 | 99% |
| 14.33 | 98% | 2.32 | 104% | 9.10 | 96% |
| 19.91 | 99% | 1.73 | 104% | 3.58 | 96% |
| 21.03 | 98% | 3.47 | 104% | 4.44 | 97% |
| 15.01 | 95% | 1.93 | 105% | 3.86 | 96% |

The results show, as desired, that charcoal absorption essentially reduces the apparent free testosterone level of patient samples, as measured by the analog procedure, to zero, that is, to percent bound values of approximately 100% B/Bₒ. Since charcoal absorption removes testosterone along with other steroids and small molecules from serum sample, while leaving larger molecules such as albumin, SHBG and other binding proteins, this experiment helps to confirm that the analog free testosterone procedure is not influenced by levels of the transport proteins as such.

Example 6: Since non-esterified free fatty acids (NEFA) have a higher association constant to albumin than does testosterone, addition of NEFA should displace free testosterone from albumin. This was confirmed by an experiment in which various amounts of oleic acid were added to each of three patient samples. The effects on the apparent free testosterone levels are shown in Table 24.

**Table 25.**

| Oleic Acid Added | Patient 1 | Patient 2 | Patient 3 | Mean |
|---|---|---|---|---|
| 0 mmol/l | 6.2 | 3.3 | 10.0 | 6.5 |
| 2.5 | 7.3 | 4.7 | 11.6 | 7.9 |
| 5.0 | 11.6 | 11.1 | 21.8 | 14.8 |
| 7.5 | 21.2 | 16.0 | 32.3 | 23.2 |
| 10.0 | 30.3 | 17.9 | 47.8 | 32.0 |

## Claims

1. A method for measuring the concentration of free testosterone ligand in a biological fluid in the presence of bound ligand and endogenous binding proteins, without disturbing the equilibrium between free ligand and protein-bound ligand, which method comprises
(a) incubating, in the absence of salicylate, 2,4-dinitrophenol and 8-anilino-1-naphthalenesulfonic acid, a sample of the biological fluid with (i) a ligand analog tracer which, due to its chemical structure, does not bind to some of the endogenous binding proteins but does bind to at least one other endogenous binding protein, (ii) a concentration of a specific ligand binder having an affinity constant and selectivity for the free ligand such that the equilibrium between free ligand and protein-bound ligand is not disturbed and (iii) a concentration of sulfobromophthalein (SBP) that inhibits the binding of the ligand analog tracer to said at least one other endogenous binding protein sufficient to block reaction between the ligand analog tracer and said at least one other endogenous binding protein without displacing ligand from protein-bound ligand;
(b) separating the ligand analog tracer bound to the specific ligand binder from unbound tracer; and
(c) determining the concentration of free ligand in said biological fluid.

2. A method according to claim 1 wherein, in step (c), the concentration of free ligand in said biological fluid is determined by comparing the bound fraction of ligand analog tracer in said sample to the bound fraction in a given set of free ligand calibrators.

3. A method according to claim 1 or 2 wherein the specific ligand binder is an antibody to said free ligand.

4. A method according to any one of the preceding claims wherein the specific ligand binder is immobilized on a solid substrate.

5. A method according to claim 4 wherein the solid substrate is polypropylene.

6. A method according to any one of the preceding claims wherein the ligand analog tracer is labelled with at least one radioactive atom, an enzyme, fluorophor, light chromophore or chemiluminescent group.

7. A method according to claim 6 wherein the ligand analog tracer is iodinated 6-hydroxytestosterone-19-carboxymethyl ether histamine.

8. A method according to any one of the preceding claims when carried out at about 37°C and at about pH 7.4.

9. A method according to claim 2 wherein said free ligand calibrators have been prepared by adding different amounts of the ligand to ligand-free human serum, calibrating by equilibrium dialysis and assigning free ligand values.

## Patentansprüche

1. Verfahren zum Messen der Konzentration von freiem Testosteron-Liganden in einem biologischen Fluid in Gegenwart von gebundenem Liganden und von endogenen Bindeproteinen, ohne Störung des Gleichgewichts zwischen freiem Liganden und Protein-gebundenem Liganden, wobei das Verfahren folgendes umfaßt:
(a) Inkubieren, in Abwesenheit von Salicylat, von 2,4-Dinitrophenol und 8-Anilino-1-naphthalinsulfonsäure, einer Probe des biologischen Fluids mit (i) einem ligandenanalogen Tracer, welcher, infolge seiner chemischen Struktur, an einige der endogenen Bindeproteine nicht bindet, aber an mindestens ein anderes endogenes Bindeprotein bindet, (ii) einer Konzentration eines spezifischen Ligandenbindestoffs mit einer Affinitätskonstante und Selektivität für den freien Liganden in einer Weise, daß das Gleichgewicht zwischen freiem Liganden und Protein-gebundenem Liganden nicht gestört wird, und (iii) einer Konzentration an Sulfobromphthalein (SBP), die die Bindung des ligandenanalogen Tracers an das mindestens eine weitere endogene Bindeprotein ausreichend verhindert, um die Reaktion zwischen dem ligandenanalogen Tracer und dem mindestens einen weiteren endogenen Bindeprotein zu blockieren, ohne den Liganden aus Protein-gebundenem Ligand zu verdrängen;
(b) Trennen des an den spezifischen Ligandenbindestoff gebundenen ligandenanalogen Tracers von ungebundenem Tracer; und
(c) Bestimmen der Konzentration an freiem Liganden in dem biologischen Fluid.

2. Verfahren nach Anspruch 1, wobei in Schritt (c) die Konzentration an freiem Liganden in dem biologischen Fluid durch Vergleichen des gebundenen Anteils des Ligandenanalog-Tracers in der Probe mit dem gebundenen Anteil in einem vorgegebenen Satz von Kalibratoren für den freien Liganden festgestellt wird.

3. Verfahren nach Anspruch 1 oder 2, wobei der spezifische Ligandenbindestoff ein Antikörper zu dem freien Liganden ist.

4. Verfahren nach mindestens einem der vorstehenden Ansprüche, wobei der spezifische Ligandenbindestoff auf einem festen Substrat immobilisiert ist.

5. Verfahren nach Anspruch 4, wobei das feste Substrat Polypropylen ist.

6. Verfahren nach mindestens einem der vorstehenden Ansprüche, wobei der ligandenanaloge Tracer mit mindestens einem radioaktiven Atom, einem Enzym, Fluorophor, Licht-Chromophor oder einer Chemolumineszenzgruppe markiert ist.

7. Verfahren nach Anspruch 6, wobei der ligandenanaloge Tracer iodiertes 6-Hydroxytestosteron-19-carboxymethyletherhistamin ist.

8. Verfahren nach mindestens einem der vorstehenden Ansprüche, das bei etwa 37°C und bei einem pH-Wert von etwa 7,4 durchgeführt wird.

9. Verfahren nach Anspruch 2, wobei die Kalibratoren für den freien Liganden durch Zusetzen unterschiedlicher Mengen des Liganden zu ligandenfreiem menschlichen Serum, Kalibrieren durch Gleichgewichtsdialyse und Zuweisen von Werten des freien Liganden hergestellt wurden.

## Revendications

1. Une méthode pour mesurer la concentration du ligand testostérone libre dans un fluide biologique en présence de ligand lié et de protéines de liaison endogènes, sans rompre l'équilibre entre le ligand libre et le ligand lié aux protéines, méthode qui comprend les étapes consistant à
(a) incuber, en l'absence de salicylate, 2,4-dinitrophénol et acide 8-anilino-1-naphtalène-sulfonique, un échantillon du fluide biologique avec (i) un traceur analogue du ligand qui, en raison de sa structure chimique, ne se lie pas à certaines des protéines de liaison endogènes mais se lie à au moins une autre protéine de liaison endogène, (ii) une concentration d'un lieur spécifique du ligand ayant une constante d'affinité et une sélectivité pour le ligand libre telles que l'équilibre entre le ligand libre et le ligand lié aux protéines n'est pas rompu et (iii) une concentration de sulfobromophtaléine (SBP) qui inhibe la liaison du traceur analogue du ligand à au moins ladite autre protéine de liaison endogène suffisamment pour bloquer la réaction entre le traceur analogue du ligand et au moins ladite autre protéine de liaison endogène sans déplacer le ligand du ligand lié aux protéines ;
(b) séparer le traceur analogue du ligand lié au lieur spécifique du ligand du traceur non lié ; et
(c) déterminer la concentration de ligand libre dans ledit fluide biologique.

2. Une méthode selon la revendication 1 dans laquelle, à l'étape (c), la concentration de ligand libre dans ledit fluide biologique est déterminée en comparant la fraction liée du traceur analogue du ligand dans ledit échantillon à la fraction liée dans un ensemble donné d'étalons de ligand libre.

3. Une méthode selon la revendication 1 ou 2 dans laquelle le lieur spécifique du ligand est un anticorps dirigé contre ledit ligand libre.

4. Une méthode selon l'une quelconque des revendications précédentes dans laquelle le lieur spécifique du ligand est immobilisé sur un substrat solide.

5. Une méthode selon la revendication 4 dans laquelle le substrat solide est le polypropylène.

6. Une méthode selon l'une quelconque des revendications précédentes dans laquelle le traceur analogue du ligand est marqué avec au moins un atome radioactif, une enzyme, un fluorophore, un chromophore lumineux ou un groupe chimioluminescent.

7. Une méthode selon la revendication 6 dans laquelle le traceur analogue du ligand est le 6-hydroxytestostérone-19-carboxyméthyléther d'histamine iodinée.

8. Une méthode selon l'une quelconque des revendications précédentes qui est effectuée à environ 37°C et à environ pH 7,4.

9. Une méthode selon la revendication 2 dans laquelle lesdits étalons de ligand libre ont été préparés en ajoutant différentes quantités de ligand à du sérum humain exempt de ligand, en étalonnant par dialyse à l'équilibre et en assignant des valeurs de ligand libre.
